# EUROPEAN PATENT APPLICATION

(11) **EP 4 495 123 A1**
(43) Date of publication of application: **22.01.2025**
(21) Application number: 23186273.1
(22) Date of filing: 19.07.2023
(51) Int. Cl.: C07D 495/04

(54) **AN IMPROVED PROCESS FOR PRODUCING D-BIOTIN INTERMEDIATES**

(71) Applicant: DSM IP Assets B.V., 6221 BE Maastricht (NL)
(72) Inventor: MUELLER, Marc-André, 4303 Kaiseraugst (CH); GOY, Roman, 4303 Kaiseraugst (CH); ZIMMERMANN, Viktor, 4303 Kaiseraugst (CH); WAECHTER, Ralph, 4303 Kaiseraugst (CH); MÜLLER, Thomas, 4303 Kaiseraugst (CH); BONRATH, Werner, 4303 Kaiseraugst (CH)
(74) Representative: dsm-firmenich IP

(57) **Abstract**

The present invention provides a process for producing a compound of formula (I), comprising catalytic hydrogenation of the compound of formula (II) to obtain the compound of formula (I), wherein the compound of formula (II) is subject to nanofiltration before the hydrogenation. According to the process of the present invention, catalytic poison can be significantly reduced. 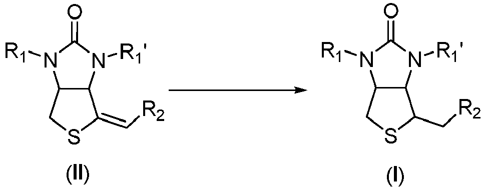 wherein R₁ and R₁' are independently H, lower alkyl, aryl or acyl group, optionally substituted by one or more substituents; and R₂ is lower alkyl optionally substituted by lower alkoxyl or carboxyl group.

## Description

### Technical Field

The present invention is related to a process for the preparation of D-biotin intermediates.

### Background of the Invention

The D-Biotin, also known as Vitamin H, is mainly applied to the fields of medicine and sanitation, nutrition enhancer, feed additive, cosmetics and drinks, etc. The molecular structural formula of the D-Biotin is shown as follows:

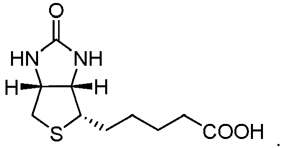

Since the debut of industrially synthetized D-biotin of a Swiss company Roche in 1949, the synthesis methods have been still undergone many researches in the world. To date, many about total synthesis routes have been reported. Yet, the most industrial process for D-biotin uses thiolactone compounds (a) to produce the intermediate compound (b) which is then converted by catalytic hydrogenation to the compound (c) and finally to the D-biotin. In the step of catalytic hydrogenation, 40 atmosphere of hydrogen pressure and 10wt% of Raney-nickel catalyst were used (see US 3,740,416 A).

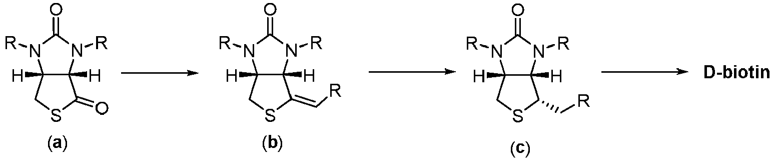

An improved process of the catalytic hydrogenation of the compound (b) uses 5wt% palladium on carbon and 50 bar of hydrogen pressure. However, the yield of the compound (c) was lower to 72% (see US 4,876,350 A).

In addition, all the above-mentioned processes have the problem of catalytic poison, which limits the efficiency of the reaction and make high pressure and high catalyst dosage necessary in the reaction (see Masahiko Seki et al., Chem. Eur. J., 2004, 10, 6102 - 6110).

Therefore, there is still demand in an improved process for producing the intermediate compound of biotin in the industry.

### Summary of the Invention

The present invention provides an improved process for producing an intermediate compound of formula (I), comprising catalytic hydrogenation of the compound of formula (II) to obtain the compound of formula (I), wherein the compound of formula (II) is subject to nanofiltration before the hydrogenation. 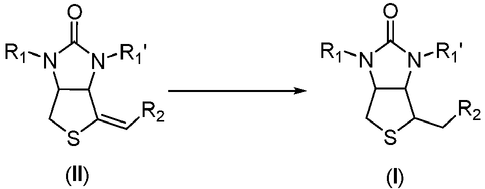 wherein R₁ and R₁' are independently H, lower alkyl, aryl or acyl group, optionally substituted by one or more substituents; and R₂ is lower alkyl optionally substituted by lower alkoxyl or carboxyl group.

According to the process of the present invention, catalytic poison can be significantly reduced. As a result, the compound of formula (II) can be hydrogenated into the compound of formula (I) with a high yield while the dosage of the catalyst can be reduced, so the process of the present invention is more cost-efficient compared to prior arts.

### Detailed description of the Invention

In the present invention, the term "lower alkyl" as used refers to C₁-C₁₀ alkyl, i.e., branched or unbranched, cyclic or non-cyclic, saturated hydrocarbon comprising 1-10 carbon atoms. Preferably, the "lower alkyl" is C₁-C₆ alkyl, including but not limited to methyl, ethyl, propyl, isopropyl, cyclopropyl, butyl, isobutyl, tert-butyl, cyclobutyl, pentyl, iso-pentyl, tert-pentyl, cyclopentyl, hexyl, isohexyl, tert-hexyl and cyclohexyl. More preferably, the "lower alkyl" is methyl or ethyl.

In the present invention, the term "aryl" as used refers to aromatic hydrocarbon such as substituted and unsubstitued phenyl, benzyl, xylyl and naphthalenyl.

In the present invention, the term "acyl group" as used refers to the structure R'-C(=O)-, wherein R' is H or lower alkyl.

In the present invention, the term "lower alkoxyl" as used refers to the structure represented by (lower alkyl)-O-, wherein the "lower alkyl" is defined as above.

In the present invention, the term "carboxyl group" as used refers to the structure -C(=O)OH.

In the present invention, the term "halo" or "halogen" refers to a group of elements including fluorine (F), chlorine (CI), bromine (Br) and iodine (I).

In the present invention, the term "substituent" or "substituents" as used refers to C₁-C₆ alkyl, C₁-C₆ alkoxyl, C₁-C₆ alkylthio, aryl, hydroxyl, halo, halide, -NH₂, -NO₂, cyano and/or isocyano.

The inventor of the present invention surprisingly discovered that, with additional pre-treatment as described herein, the compound of formula (I) can be hydrogenated in a high yield while the catalyst is used in a lower amount.

Particularly, the present invention provides a process for producing a compound of formula (I), comprising catalytic hydrogenation of the compound of formula (II) to obtain the compound of formula (I), wherein the compound of formula (II) is subject to nanofiltration before the hydrogenation. 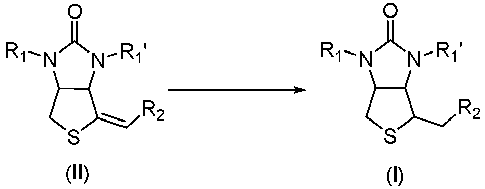 wherein R_{1,} R₁' and R₂ are independently defined as above.

Preferably, R₁ and R₁' are independently H, C₁-C₆ alkyl, aryl or C₁-C₆ acyl group, optionally substituted by one or more substituents. More preferably, R₁ and R₁' are independently H, methyl, ethyl, propyl, butyl, phenyl, benzyl, formyl, acetyl, optionally substituted by one or more substituents. The most preferably, R₁ and R₁' are independently H, benzyl or acetyl, optionally substituted by one or more substituents.

Preferably, R₂ is C₁-C₆ alkyl optionally substituted by C₁-C₆ alkoxyl or carboxyl group. More preferably R₂ is methyl, ethyl, propyl or butyl, optionally substituted by methoxyl or ethoxyl, or carboxyl group. The most preferably, R₂ is ethyl or propyl substituted by methoxyl or carboxyl group.

In one embodiment, R₁ and R₁' are independently H, benzyl or acetyl; and R₂ is ethyl or propyl substituted by methoxyl.

In another embodiment, R₁ and R₁' are independently H, benzyl or acetyl; and R₂ is ethyl or propyl substituted by carboxyl group.

In the process of the present invention, the nanofiltration is conducted with nanofiltration membranes which have molecular weight cutoff (MWCO) of from 100 to 1000, preferably from 200 to 800, more preferably from 250 to 600. Preferably, the nanofiltration membranes are selected from polymeric membranes and ceramic membranes. More preferably, the nanofiltration membranes are polymeric. Examples of the commercialized membranes are BORSIG OSN Membranes oNF-1, oNF-2 and oNF-3 (Borsig Membrane Technology GmbH, Germany), and Evonik under the trade name PURAMEM^{®}.

In the process of the present invention, the nanofiltration is carried out in an organic solvent. The organic solvent may be selected from the group consisting of alcohols such as methanol and ethanol, esters such as ethyl acetate (EtOAc), and aromatic hydrocarbons such as toluene. Preferably, the organic solvent is toluene.

It is preferred that the nanofiltration in the process of the present invention is performed in a cross-flow mode. It is further preferred that the nanofiltration is performed in a diafiltration mode.

In the process of the present invention, the nanofiltration may be carried out at the temperature of 0°C to 50°C, preferably from 10°C to 30°C, more preferably at room temperature. It is preferred that the nanofiltration is carried out at an elevated pressure, usually and preferably the pressure applied is from 5 bar to 50 bar, more preferably from 10 bar to 40 bar such as 20 bar.

In the process of the present invention, the obtained permeate from the nanofiltration can be used into catalytic hydrogenation directly or after removing the solvent. Preferably, the compound of formula (II) is isolated from the permeate before the hydrogenation. The obtained retentate from the nanofiltration can be discarded or re-fed into the nanofiltration. Preferably, the retentate is re-fed to the nanofiltration.

In the process of the present invention, the catalytic hydrogenation of the compound of formula (II) may be carried out according to the disclosure of prior arts such as US 3,740,416 A and US 4,876,350 A.

In the process of the present invention, the catalyst used in the catalytic hydrogenation is preferably selected from the group consisting of palladium, palladium hydroxide, nickel, nickel-alloy, platinum, ruthenium and/or rhodium on the usual support materials such as carbon, clay, etc.. More preferably, the catalyst is selected from the group consisting of palladium, palladium hydroxide and nickel-alloy (e.g. Raney-nickel) in the presence or absence of a modifier (e.g. formic acid, formaldehyde, carbon monoxid).

In the process of the present invention, the catalyst is used in an amount of from 0.01wt% to 1wt%, preferably from 0.05wt% to 0.8wt%, more preferably from 0.1wt% to 0.5wt% such as 0.1wt%, 0.12wt%,0.15wt%, 0.2wt%, 0.25wt%, 0.3wt%, 0.35wt%, 0.4wt%, 0.45wt%, 0.5wt% and 0.6wt% (calculated by the metal or metal hydroxide contained in the catalyst) based on the weight of the compound of formula (II).

In the process of the present invention, the compound of formula (II) is preferably produced by Grignard reaction of the compound of formula (III). 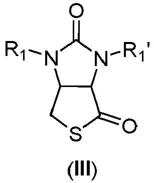 Wherein R₁ and R₁' are independently defined as above.

The Grignard reaction of the compound of formula (III) can be carried out according to the process known the art, such as those disclosed in US 3,740,416 A and US 5,847,152 A.

According to the process of the present invention, the compound of formula (II) can be hydrogenated into the compound of formula (I) with a high yield, even in a lower dosage of the catalyst, so the process of the present invention is more cost-efficient compared to prior arts.

Based on the good results of the process of the present invention, it is anticipated by a person skilled in the art that the catalytic poison in the hydrogenation should result from impurities contained in the raw material of the compound of the formula (I), instead of the sulfide moiety of the compound of formula (II). Accordingly, the process of the present invention is also suitable to use a stored material of the compound of formula (II) which contains the impurities.

In addition, the process of the present invention can be run in a continuous way.

The present invention also provides a process for producing (+)-biotin which comprises the process for producing the compound of formula (I) as described herein.

The process of the present invention is further illustrated by the following examples.

### Examples

### General procedures

### Preparation of raw materials

In the examples according to the present invention, the raw material (3aS,6aR,E)-1,3-dibenzyl-4-(3-methoxypropylidene)tetrahydro-1H-thieno[3,4-d]imidazol-2(3H)-one ("THE") was prepared by Grignard addition according to the procedures described in US 3,740,416 A, and the raw material (E)-5-((3aS,6aR)-1,3-dibenzyl-2-oxohexahydro-4H-thieno[3,4-d]imidazol-4-ylidene)pentanoic acid **("DbBTN")** was prepared by Grignard addition according to the procedures described in US 5,847,152 A.

### Nanofiltration

The raw material (THE or DbBTN) (1 g) dissolved in an organic solvent (15 mL) was subjected to nanofiltration in a cross-flow mode during 65 hours. During the nanofiltration, a particular pressure was applied at 25 °C. After the nanofiltration, the permeate was collected and evaporated and the obtained residue was tested in the catalytic hydrogenation.

### Catalytic hydrogenation

Into an autoclave, THE (1 g) was dissolved in a MeOH (5 mL). A specific amount of catalyst Pd/C was added into the rection mixture. After purged with nitrogen, the reaction mixture was stirred at 30 bar hydrogen pressure and at 75°C. After 16 h, the reaction mixture was cooled down to room temperature, hydrogen was released, and the reactor was purged with nitrogen. After all volatiles were evaporated, the yield of THA was determined by GC.

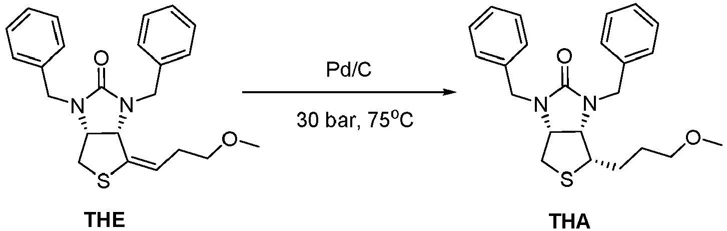

DbBTN was hydrogenated to DhBTN according to the same procedures except that the solvent was EtOH and the reaction temperature was 100°C. The yield was determined by qHPLC.

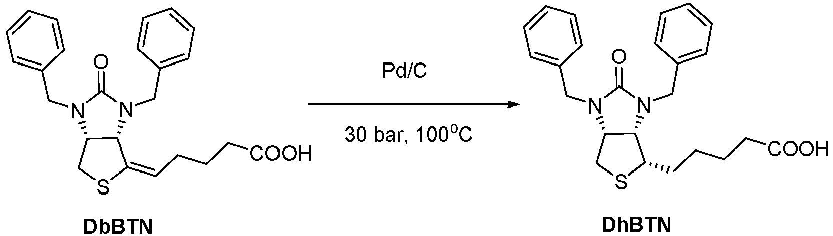

### Example 1: Catalytic hydrogenation of THE without nanofiltration pre-treatment

THE obtained from the Grignard addition was hydrogenated according to the process described above and the results are shown in Table 1.

**Table 1**

| **Entry** | **Catalyst Pd (mg)** | **THE (area%)** | **THA (area%)** |
|---|---|---|---|
| 1 | 1.2 | 91.7 | 5.9 |
| 2 | 2.0 | 81.6 | 15.8 |
| 3 | 2.8 | 45.4 | 51.7 |
| 4 | 3.2 | 19.2 | 78.1 |
| 5 | 4.0 | 0.8 | 96.4 |

### Example 2: Catalytic hydrogenation of THE with nanofiltration pre-treatment

With the same THE as Example 1, nanofiltration was carried out and then the obtained THE was hydrogenated with Pd/C (1.2 mg Pd per 1 g THE) according to the process described above. The condition of nanofiltration and the results of hydrogenation are shown in Table 2.

**Table 2**

| **Entry** | **Nanofiltration** | | | **Hydrogenation** | |
|---|---|---|---|---|---|
| | **Membrane (MWCO)** | **Solvent (mL)** | **P (bar)** | **THE (area%)** | **THA (area%)** |
| 1 | Borsig oNF-1 (600) | EtOAc (150) | 10 | 41.0 | 56.9 |
| 2 | Borsig oNF-1 (600) | Toluene (150) | 10 | 3.6 | 94.8 |
| 3 | Borsig oNF-2 (350) | EtOAc (150) | 10 | 50 | 47.5 |
| 4 | Borsig oNF-2 (350) | Toluene (150) | 10 | 26.3 | 71.8 |
| 5 | Evonik PuraMen Selective (n.d.) | Toluene (150) | 10 | 54.8 | 42.7 |
| 6 | Evonik PuraMen 280 (280) | Toluene (150) | 20 | 59.8 | 38.0 |

| | | | | | |
|---|---|---|---|---|---|
| n.d.= not defined in product data sheets. | | | | | |

### Example 3: Catalytic hydrogenation of permeate THE and retentate THE from the nanofiltration

THE obtained from the Grignard addition was subjected to nanofiltration by using a Borsig oNF-1 in toluene at room temperature under 10 bar. Then the permeate THE and the obtained retentate were separately hydrogenated with Pd/C (1.2 mg Pd per 1 g THE) according to the procedure described above. The results of hydrogenation are shown in Table 3.

**Table 3**

| **Entry** | **THE** | **THE (area%)** | **THA (area%)** |
|---|---|---|---|
| 2 | Permeate THE | 0 | 98.6 |
| 3 | Retentate THE | 95.7 | 0 |

### Example 4: Catalytic Hydrogenations of DhBTN.

DhBTN prepared by Grignard addition was hydrogenated with Pd/C without and with nanofiltration pre-treatment according to the procedures described above. The nanofiltration of DhBTN was runed by using Borsig oNF-1 in toluene at room temperature under 10 bar. The results are shown in Table 4.

**Table 4**

| **Entry** | **DhBTN** | **Catalyst Pd (mg)** | **DhBTN (%)** | **Yield of DbBTN (%)** |
|---|---|---|---|---|
| 1 | No nanofiltration | 0.4 | 0 | >99 |
| 2 | No nanofiltration | 0.29 | 53 | 47 |
| 3 | No nanofiltration | 0.22 | 78 | 22 |
| 4 | After nano-filtration | 0.25 | 0 | >99 |

## Claims

1. A process for producing a compound of formula (I), comprising catalytic hydrogenation of the compound of formula (II) to obtain the compound of formula (I), wherein the compound of formula (II) is subject to nanofiltration before the hydrogenation. 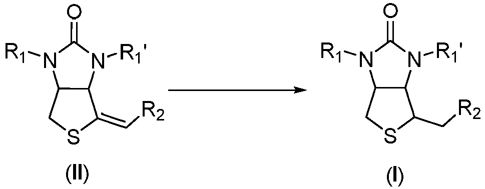 wherein R₁ and R₁' are independently H, lower alkyl, aryl or acyl group, optionally substituted by one or more substituents; and R₂ is lower alkyl optionally substituted by lower alkoxyl or carboxyl group.

2. The process of claim 1, wherein the nanofiltration is conducted with nanofiltration membranes which have molecular weight cutoff (MWCO) of from 100 to 1000, preferably from 200 to 800, more preferably from 250 to 600.

3. The process of claim 1, wherein the nanofiltration membranes are selected from polymeric membranes and ceramic membranes, preferably selected from the group consisting of BORSIG OSN Membranes oNF-1, oNF-2 and oNF-3 (Borsig Membrane Technology GmbH, Germany), and Evonik under the trade name PURAMEM^{®}.

4. The process of any one of claims 1 to 3, wherein the nanofiltration is carried out in an organic solvent selected from the group consisting of alcohols such as methanol and ethanol, esters such as ethyl acetate (EtOAc), and aromatic hydrocarbons such as toluene.

5. The process of any one of claims 1 to 3, wherein the nanofiltration is performed in a cross-flow mode.

6. The process of any one of claims 1 to 5, wherein the nanofiltration is performed in a diafiltration mode.

7. The process of any one of claims 1 to 6, wherein the catalyst used in the catalytic hydrogenation is preferably selected from the group consisting of palladium, palladium hydroxide, nickel, nickel-alloy, platinum, ruthenium and/or rhodium on the usual support materials such as carbon, clay, etc..

8. The process of any one of claims 1 to 6, wherein the catalyst is used in the catalytic hydrogenation in an amount of from 0.01wt% to 1wt%, preferably from 0.05wt% to 0.8wt%, more preferably from 0.1wt% to 0.5wt% such as 0.1wt%, 0.12wt%,0.15wt%, 0.2wt%, 0.25wt%, 0.3wt%, 0.35wt%, 0.4wt%, 0.45wt%, 0.5wt% and 0.6wt% (calculated by the metal or metal hydroxide contained in the catalyst) based on the weight of the compound of formula (II).

9. The process of any one of claims 1 to 8, wherein the compound of formula (II) is preferably produced by Grignard reaction of the compound of formula (III). 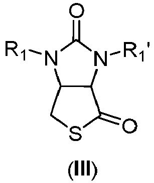 wherein R₁ and R₁' are independently defined as claim 1.

10. The process of any one of claims 1 to 9, wherein the process is run in a continuous way.

11. A process for producing (+)-biotin which comprises the process for producing the compound of formula (I) according to any one of claims 1 to 10.
